(19) Europäisches Patentamt
European Patent Office
Office européen des brevets

(11) **EP 2 979 609 A1**

(12) **EUROPEAN PATENT APPLICATION**
published in accordance with Art. 153(4) EPC

(43) Date of publication:
**03.02.2016 Bulletin 2016/05**

(51) Int Cl.:
***A61B 1/00*** *(2006.01)*   ***G02B 23/24*** *(2006.01)*

(21) Application number: **14772996.6**

(86) International application number:
**PCT/JP2014/051200**

(22) Date of filing: **22.01.2014**

(87) International publication number:
**WO 2014/156242 (02.10.2014 Gazette 2014/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.03.2013   US 201361806459 P**

(71) Applicant: **Olympus Corporation**
**Shibuya-ku**
**Tokyo 151-0072 (JP)**

(72) Inventor: **YOSHIMURA, Katsuhiko**
**Tokyo 151-0072 (JP)**

(74) Representative: **Schicker, Silvia**
**Wuesthoff & Wuesthoff**
**Patentanwälte PartG mbB**
**Schweigerstraße 2**
**81541 München (DE)**

(54) **MANIPULATOR, MANIPULATOR SYSTEM, AND MANIPULATOR CONTROL METHOD**

(57)    An object of the invention is to provide a manipulator capable of relieving less experienced operators of uncomfortableness.

As shown in Fig. 11, the manipulator 1 comprises an insert unit 2 capable of being inserted into the body cavity, an operating unit 3 for operation of the insert unit 2, an extending/retracting unit 4 for moving the insert unit 2 forward or backward, a drive unit 6 for driving the insert unit 2 and extending/retracting unit 4, and a control unit 9 for controlling the drive unit 6 in association with oper-

ation of said operating unit 3. The manipulator 1 is characterized in that the insert unit 2 includes a bendable portion 22, the drive unit 6 includes a bending movement driving portion 61 for generating driving force for bending the bendable portion 22 and an extension/ retraction driving portion 62 for generating driving force for moving the extending/retracting unit 4 forward or backward, and the control unit 9 controls the extension/retraction driving portion 61 and bending movement driving portion 62 in association with operation of the operating unit 3.

FIG.11

**Description**

TECHNICAL FIELD OF THE INVENTION

The present invention relates to a manipulator designed to be inserted into the body cavity, a manipulator system, and a manipulator control method.

BACKGROUND ART

A manipulator having an elongate insert inserted in the body cavity has been extensively used, in which a bendable portion at the front end of the insert is pulled and bent by a wire or the like for the purpose of observing organs in the body cavity and giving medical treatments to them.

For instance, Patent Publication 1 (JP(A) 2005-74148) discloses a technique including an extending/retracting member located movably in a bendable portion of an insert via a guide wherein movement of the extending/retracting member is adjusted such that it advances toward or retreats from the bendable portion of the insert whereby the bendable portion of the insert is positioned selectively, substantially linearly or in a bendable manner.

PRIOR ART PUBLICATIONS

Patent Publication 1: JP(A) 2005-74148

SUMMARY OF THE INVENTION

OBJECT OF THE INVENTION

While an operator uses a conventional manipulator to observe a site of interest and give medical treatment to it, it is often necessary for the operator to observe the site of interest or give medical treatment to it from another angle. As the operator bends the manipulator without changing position, it causes the manipulator to be bent before reaching the site of interest with the result that the site of interest comes off from the field of vision. A less experienced operator would feel something wrong with that manipulator, and could not make full use of it.

With such situations in mind, the invention has for its object to provide a manipulator, a manipulator system and a manipulator control method, which may relieve the operator of such un-comfortableness.

MEANS FOR ACHIEVING THE OBJECT

According to one embodiment, the invention provides a manipulator including an insert unit capable of being inserted through the body cavity, an operating unit for operating the insert unit, an extending/retracting unit for moving the insert unit forward or backward, a drive unit for driving the insert unit and the extending/retracting unit, and a control unit for controlling the drive unit in association with operation of the operating unit, characterized in that the insert unit includes a bendable portion, and the drive unit includes a bending movement driving portion for generating driving force for bending the bendable portion and an extension/retraction driving portion for generating driving force for moving the extending/retracting unit forward or backward, characterized in that the control unit controls the extension/retraction driving portion and the bending movement driving portion in association with operation of the operating unit.

ADVANTAGES OF THE INVENTION

With the manipulator, manipulator system and manipulator control method according to one embodiment of the invention, it is possible to relieve less experienced operators of un-comfortableness.

BRIEF EXPLANATION OF THE DRAWINGS

Fig. 1 is a diagrammatic view of the manipulator 1 according to one embodiment of the invention.
Fig. 2 is an enlarged view of the insert 2 according to one embodiment of the invention.
Fig. 3 is a schematic view of the operating unit 3 according to one embodiment of the invention.
Fig. 4 is a schematic view of the drive unit 6 according to one embodiment of the invention.
Fig. 5 is an enlarged view of the extending/retracting unit 4 according to one embodiment of the invention.

Fig. 6 is a schematic view of one example of operation of the insert 2 according to one embodiment of the invention.

Fig. 7 is a schematic view of one example of operation of the extending/retracting unit 4 according to one embodiment of the invention.

Fig. 8 is a schematic view of another example of the extending/retracting unit 4.

Fig. 9 is a schematic view of yet another example of the extending/retracting unit 4.

Fig. 10 is a diagrammatic view of operation of the manipulator 1 according to one embodiment of the invention.

Fig. 11 is a block diagram of the manipulator 1 according to one embodiment of the invention.

Fig. 12 is a schematic view of the first manual operation of the manipulator 1 in a manual mode.

Fig. 13 is a schematic view of the second manual operation of the manipulator 1 in the manual mode.

Fig. 14 is a schematic view of the third manual operation of the manipulator 1 in the manual mode.

Fig. 15 is descriptive of Equations (1) and (2) used for control of the manipulator 1 according to one embodiment of the invention in an electrically operated mode.

Fig. 16 is descriptive of Equations (3) and (4) used for control of the manipulator 1 according to one embodiment of the invention in the electrically operated mode.

Fig. 17 is a control flowchart for the manipulator 1 according to one embodiment of the invention.

Fig. 18 is a schematic view of the manipulator 1 according to one embodiment of the invention before controlled in the electrically operated mode.

Fig. 19 is a schematic view of the manipulator 1 according to one embodiment of the invention just after controlled in the electrically operated mode.

Fig. 20 is a schematic view of the manipulator 1 according to one embodiment after the completion of the electrically operated mode.

Fig. 21 is a diagrammatic view of the manipulator system 10 according to one embodiment of the invention.

Fig. 22 is a diagrammatic view of another example of the manipulator system 10.

## MODE FOR CARRYING OUT THE INVENTION

The manipulator 1 and manipulator control method according to one embodiment of the invention are now explained.

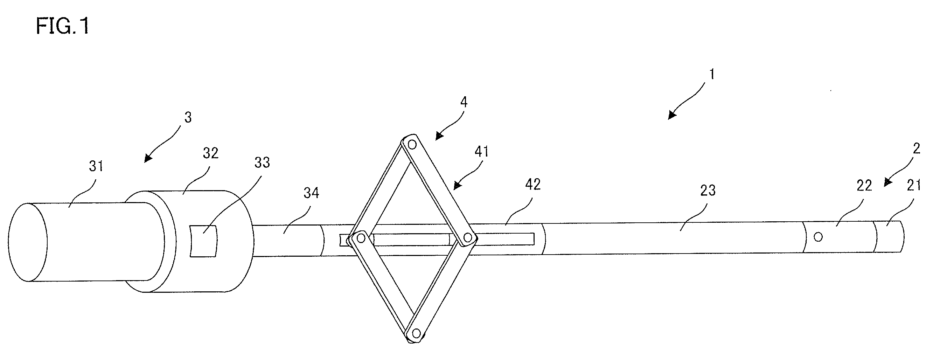

Fig. 1 is a diagrammatic view of the manipulator 1 according to one embodiment of the invention.

The manipulator 1 according to one embodiment of the invention comprises an insert unit 2, an operating unit 3, and an extending/retracting unit 4. The manipulator 1 includes the insert unit 2 on a front-end (distal end) side and the operating unit 3 on a base-end (proximal end) side, and includes the extending/retracting unit 4 on the operating unit 3 side of the insert unit 2. The insert unit 2 includes a front-end portion 21, a bendable portion 22, and a power transmission portion 23. The operating unit 3 includes a grip 31, a main body 32, a mode selection instruction portion 33, and a coupling 34. The extending/retracting unit 4 includes a link portion 41 and a guide portion 42.

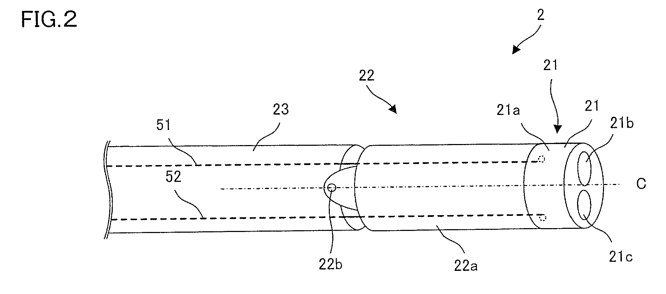

Fig. 2 is an enlarged view of the insert unit 2 of the manipulator 1 according to one embodiment of the invention.

The insert unit 2 to be inserted through the body cavity includes the front-end portion 21, bendable portion 22 and power transmission portion 23 in order from the front-end side.

The front-end portion 21 includes a cylindrical, hard main body 21a attached to the front end of the manipulator 1. Taking an endoscope as an example, the front-end portion 21 may have in it an imaging portion 21b for taking images of the site of interest, a lighting portion 21c for illuminating the site of interest, etc. A treatment tool (not shown) for giving treatment to the site of interest or the like may be built in the front-end portion 21.

The bendable portion 22 includes a substantially cylindrical block 22a to which the front-end portion 21 is attached, and a joint 22b for making a rotatable coupling between the block 22a and the power transmission portion 23 within a given angle range. The power transmission portion 23 is an elongate, substantially cylindrical shaft or like other member coupled to the front-end side to the bendable portion 22 by way of the joint 22b and on the base-end side to the extending/ retracting unit 4.

The front-end portion 21 is fixedly provided with first and second wires 51 and 52 for activation of the bendable portion. The first and second wires 51 and 52 for activation of the bendable portion are fixedly provided in opposite positions with a plane including the joint 22b in between. As an example, those positions are preferably symmetrical with respect to the center axis C of the cylindrical front-end portion 21. The first and second wires 51 and 52 for activation of the bendable portion are taken up or fed out by an actuator (not shown) or the like within the main body 32 of the operating unit shown in Fig. 1.

It is here to be noted that the structure of the insert unit 2 is not limited to this; so it may be modified in various ways. For instance, a plurality of blocks 22a and a plurality of joints 22b may be alternately combined into a bent state having a higher degree of freedom. More preferably, a set of adjoining block 22a and joint 22b in a plurality of block 22a/joints 22b combinations are rotated for each 90° about the axis of the bendable portion 22 in a straightly extended state or the center axis C of the power transmission portion 23 such that the insert unit 2 is three dimensionally movable.

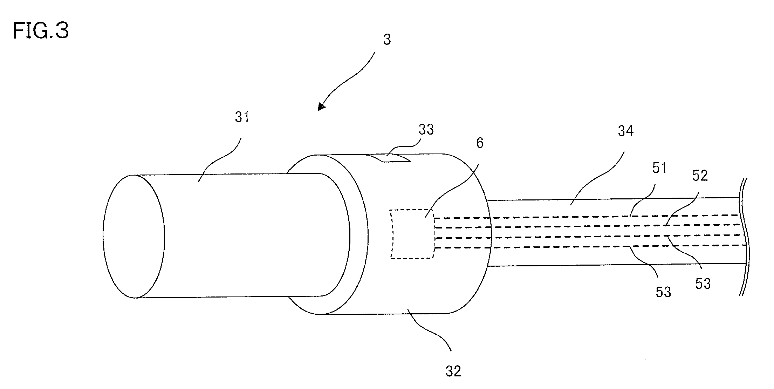

Fig. 3 is an enlarged view of the operating unit 3 according to one embodiment of the invention.

The operating unit 3 includes a grip 31 of which the operator takes a grip, a main body 32 of the operating unit 3 having a built-in drive part 6 for operating movement or the like of the insert unit 2 provided on the extending/retracting unit 4 side of the grip 31 and extending/retracting unit 4 as described later, a mode selection instruction portion 33 that can be forced in the main body 32, and a coupling 34 coupled to the extending/ retracting unit 4.

While the grip 31 here is provided in a cylindrical form, it is to be understood that it may have any desired shape provided that it is easy to grip. The main body 32 of the operating unit has the built-in drive unit 6 including a motor, a pulley and so on as described later. The main body 32 of the operating unit takes up or feeds out the first and second wires 51 and 52 for activation of the bendable portion thereby bending the bendable portion 22 of the insert unit 2 shown in Fig. 1. The main body 32 of the operating unit also takes up and feeds out a link activation wire 53 thereby causing extension and retraction of a link 41 of the extending/retracting unit 4 shown in Fig. 1.

The mode selection instruction portion 33 comprises a push switch and so on. Usually, when the push switch or the like is not pushed down, the mode selection instruction portion 33 is set in a manual mode as the first mode, and as the push switch or the like is pushed down, the mode selection instruction part 33 issues an instruction to change the manual mode over to an electrically operated mode as the second mode.

The coupling 34 has the first and second wires 51 and 52 for activation of the bendable portion and the link activation wire 53 inserted through it, and is coupled to the extending/ retracting unit 4 shown in Fig. 1.

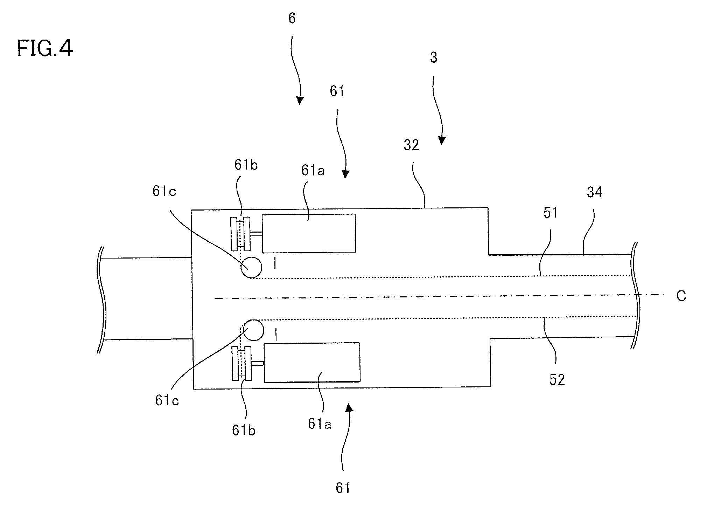

Fig. 4 is a schematic view of the drive unit 6 according to one embodiment of the invention.

The drive unit 6 includes a portion 61 for driving bending movement, and a portion 62 for driving extension/retraction (not shown). The driving portions 61 and 62 may be of the same construction; so the driving portion 61 for driving bending movement is here explained.

The driving portion 61 for driving bending movement includes a motor 61a, a driving pulley 61b, and a driven or follower pulley 61c.

One ends of the first and second wires 51 and 52 for activation of the bendable portion are firmly fixed to the main body 21a of the front-end portion 21 shown in Fig. 2, and the other ends of the first and second wires 51 and 52 are firmly fixed to the driving pulley 61b. The first and second wires 51 and 52 for activation of the bendable portion are wound around the driven pulley 61c. The driving pulley 61b is mounted on the rotary shaft of the motor 61a to take up and feed out the first and second wires 51 and 52 at the driven pulley 61b in association with the rotation of the motor 61a. In turn, the rotational driving force of the motor 61a is transmitted to the main body 21a of the front-end portion 21.

In the example of Fig. 4, two portions 61 for driving bending movement are symmetrically provided with respect to the center line C of the coupling 34 for the purpose of explaining movement in a two-dimensional plane; however, it is to be understood that the invention is not always limited to two portions 61; there may be one or two or more portions 61 provided depending on the direction of rotation of the joint 22b. For instance, when one driving portion 61 for driving bending movement is provided in combination with one wire for activation of the bendable portion, one wire for activation of the bendable portion may be looped around the driving pulley of the portion 61 for driving bending movement and the pulley mounted on the joint 22b. Then, the driving pulley is rotated by the rotational driving of one motor so that the block 22a can be bent vertically by way of the wire for activation of the bendable portion and the pulley. For three-dimensional movement, additional portions 61 for driving bending movement may be provided before and after the drawing sheet.

It is to be understood that the structure of the portion 61 for driving bending movement is not always limited to the aforesaid one; it may be modified in various ways. For instance, the respective joints 22b may be individually operated. The individually operable structure is preferable because there is an increased degree of freedom in the posture of the bendable portion 22. While the portion 61 for driving bending movement is located within the operating unit 3 in the example shown in Fig. 4, it is to be noted that it may be provided separately from the operating unit 3. That is, while the insert unit 2 is positioned near the patient, the operating unit 3 may be operated by the operator from a remote position.

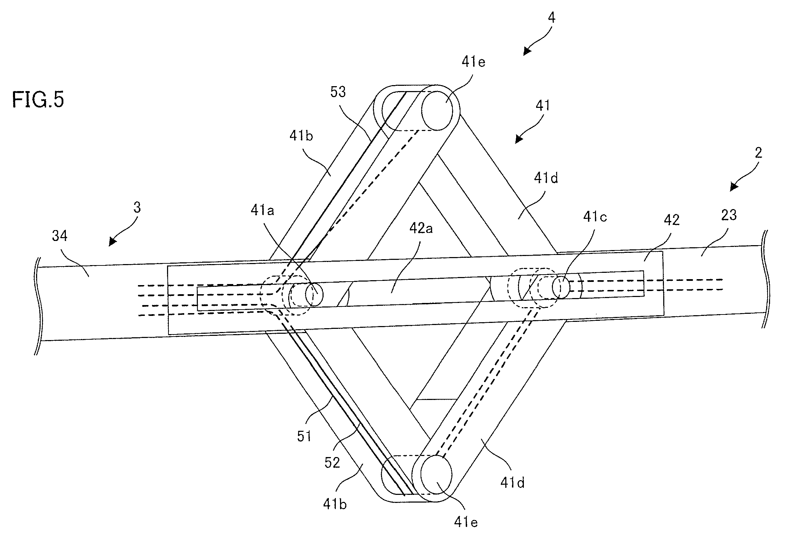

Fig. 5 is an enlarged view of the extending/ retracting unit 4 according to one embodiment of the invention.

The extending/retracting unit 4 includes a link 41 coupled on one side to the operating unit 3 and on the other side to the insert unit 2, and a guide 42 for guiding operation of the link 41.

The link 41 includes a first protrusion 41a that is mounted on the coupling 34 and guided by the guide 42, a first arm 41b that is rotatably mounted on the first protrusion 41a, a second protrusion 41c that is attached to the power transmission portion 23 and guided by the guide 42, a second arm 41d that is rotatably mounted on the second protrusion 41c, and a connecting portion 41e for making a connection between the first and second arms 41b and 41d.

The guide 42 is an elongate member that is fixed on one side to the coupling 34 and on the other side to the power transmission portion 23. The guide 42 is provided with a long opening 42a for receiving the first and second protrusions 41a and 41c. In other words, the first and second protrusions 41a and 41c are movable along the elongate opening 42a.

In this embodiment, two first arms 41b, two second arms 41d and two connecting portions 41e are provided with the guide 42 between them. The link activation wire 53 is wound around at least one connecting portion 41e, and the main body 32 of the operating unit takes up the link activation wire 53 thereby extending and retracting the link 41 having a so-called pantograph structure.

There is a cavity inside the first and second arms 41b and 41d, through which the first and second wires 51 and 52 for activation of the bendable portion, the link activation wire 53, wirings for electronic components, etc. are passed.

It is here to be noted that multiple links 41 and multiple guides 42 may be provided to make the extending/retracting distance long.

Movement of the manipulator 1 is now explained. First, one example of bending movement is explained.

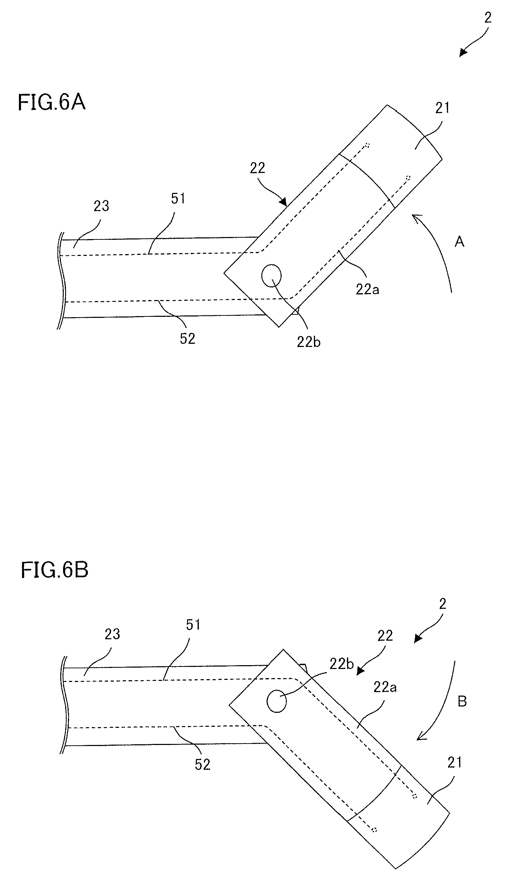

Fig. 6 is a schematic view of one example of operation of the insert unit 2 according to one embodiment of the invention.

The manipulator 1 according to one embodiment of the invention actually operates in a three-dimensional space; for the sake of an easy understanding of the invention, however, operation of the manipulator 1 in a two-dimensional plane is here explained with reference to a schematic view like Fig. 6.

Before activation of the insert unit 2, the front-end portion 21, bendable portion 22, and power transmission portion 23 are in a linear state as shown in Fig. 1. As the drive unit 6 is activated by operation of the operating unit 3 shown in Fig. 3, it causes the front-end portion 21 and block 22a to be pulled by the first or second wire 51 or 52 for activation of the bendable portion so that the front-end portion 21 and bendable portion 22 bend at the joint 22b with respect to the power transmission portion 23.

Fig. 6A shows that the first wire 51 for activation of the bendable portion is being pulled by the drive unit 6 shown in Fig. 4; the front-end portion 21 and block 22a are rotated in a direction indicated by an arrow A. It is here preferable that the second wire 52 for activation of the bendable portion is fed out. Fig. 6B shows that the second wire 52 for activation of the bendable part is being pulled by the drive unit 6 shown in Fig. 4; the front-end portion 21 and block 22a are rotated in a direction indicated by an arrow B. It is here preferable that the first wire 51 for activation of the bendable portion is fed out.

It is here to be noted that for a structure including multiple blocks 22a and joints 22b, the respective joints 22b may be individually actuated. Such individual actuation is preferable because there is an increased degree of freedom in posture. The main body 32 of the operating unit may be provided separately from the operating unit 3: the main body 32 may be positioned near the patient while the operating unit 3 may be remote controlled from a remote position.

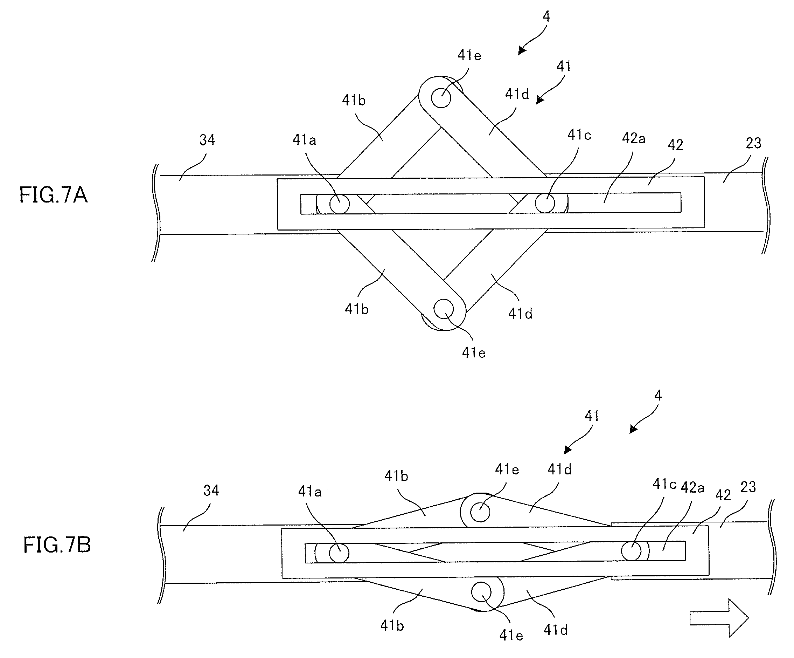

Fig. 7 is a schematic view of an example of movement of the extending/retracting unit 4 according to this embodiment of the invention.

In the embodiment of the invention here, a pantograph mechanism is used as an example of the extending/retracting unit for generating extending/retracting movement of the insert unit 2 of the manipulator 1.

As shown in Fig. 5, the link activation wire 53 is wound around the connecting portion 41e of the link 41. The link 41 is actuated by driving a motor (not shown) for taking up and feeding out the link activation wire 53.

Referring to the extending/retracting unit 4 in a state shown in Fig. 7A, as the motor (not shown) is driven to take up the link activation wire 53, it causes the connecting portion 41e to be pulled by the link activation wire 53 so that the first arm 41b rotates about the first protrusion 41a. This in turn causes the second arm 41d having the link formed on it to rotate about the second protrusion 41c.

As a result, an angle formed between the first 41b and the second arm 41d grows large, as shown in Fig. 7B, so that the second protrusion 41c moves away from the first protrusion 41a along the long opening 42a. Correspondingly, the distance between the first 41a and the second protrusion 41c becomes long, resulting in extension of the extending/retracting unit 4.

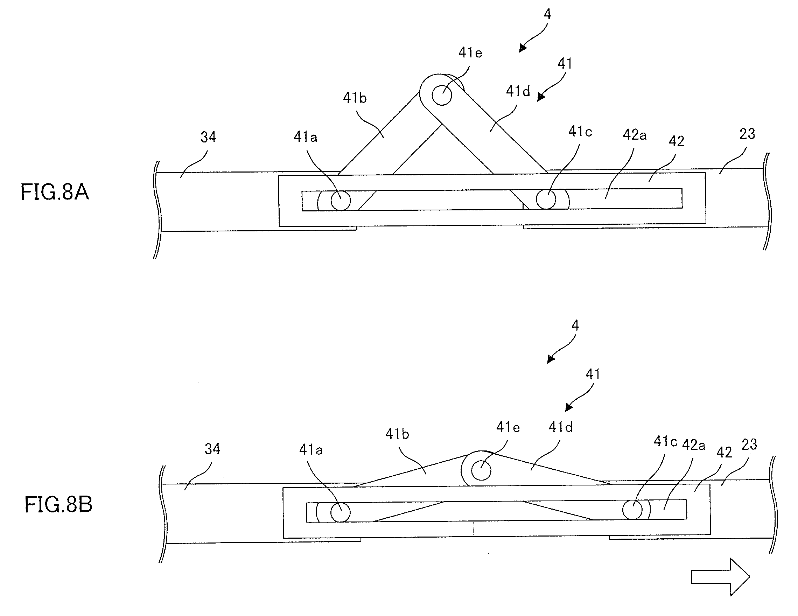

Fig. 8 is a schematic view of another example of the extending/retracting unit 4.

The extending/retracting unit 4 shown in Fig. 8 includes one first arum 41b, one second arm 41d, and one connecting portion 41e on one side alone. Such construction may contribute to weight reductions.

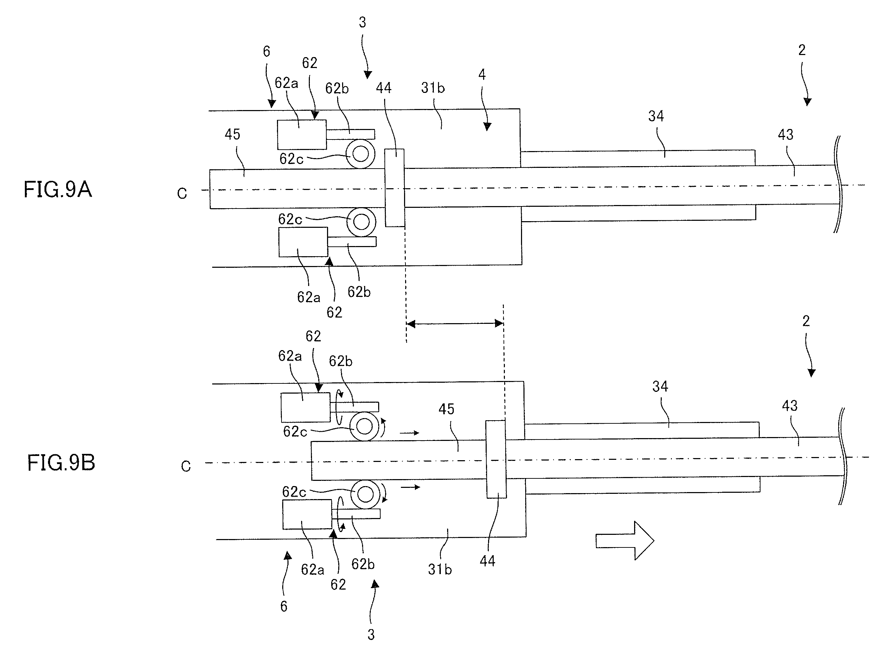

Fig. 9 is a schematic view of yet another example of the extending/retracting unit 4.

The extending/retracting unit 4 shown in Fig. 9 comprises a driving mechanism 6 as an example of the mechanism for generating extending/retracting movement of the insert unit 2 of the endoscope 1, wherein a gear mechanism is located as an extending/retracting movement driving portion 62 in the operating unit 3. The extending/ retracting unit 4 includes a movable portion 43, a stopper 44, and a pinion 45.

Located in the operating unit 3, the extending/retracting movement driving portion 62 includes a motor 62a rotated by a power source (not shown), and a motor output shaft 62b for producing the driving force of the motor 62a. A gear portion 62c meshes with the motor output shaft 62b and pinion 45 to transmit the rotations of the motor output shaft 62b from the pinion 45 to the insert unit 2 via the movable portion 43 so that the extending/retracting unit 4 and insert unit 2 advance toward or retreat from the operating unit 3.

The stopper 44 is larger in diameter than the movable portion 43 to limit movement of the movable portion 43, and should preferably be larger in diameter than the coupling 34 of the operating unit 3. The pinion 45 converts the rotational motion of the motor output shaft 62b into linear motion and transmits it to the insert unit 2 so that the movable portion 43 advances toward or retreats from the operating unit 3.

In a state shown in Fig. 9A, the movable portion 43 goes back to the coupling 34 where an exposed part of the movable portion 43 becomes short in length. In a state shown in Fig. 9B, as the extending/retracting movement driving portion 62 is actuated from this state, it first causes rotation of the motor output shaft 62b by the driving force of the motor 62a. In turn, the gear portion 62c in mesh with the motor output shaft 62b rotates, and the pinion 23c in mesh with the gear portion 62c advances.

In the example shown in Fig. 9, two extending/retracting movement driving portions 62 are symmetrically located with respect to the center line C of the movable portion 43 for the sake of an illustration of motion in the two-dimensional plane. However, it is to be noted that one or more such driving portions 62 may be provided.

It is also to be appreciated that the structure of the extending/retracting movement driving portion 62 is not limited to the examples shown in Figs. 7, 8 and 9; various modifications may be made to it. For instance, the extending/retracting movement driving portions 62 are provided within the operating unit 3 in the examples shown in Figs. 7, 8 and 9; however, they may be provided separately from the operating unit 3. That is, the extending/retracting movement driving portions 62 may be positioned near the patient and the operating unit 3 may be controlled from a remote position.

How such manipulator 1 operates is now explained.

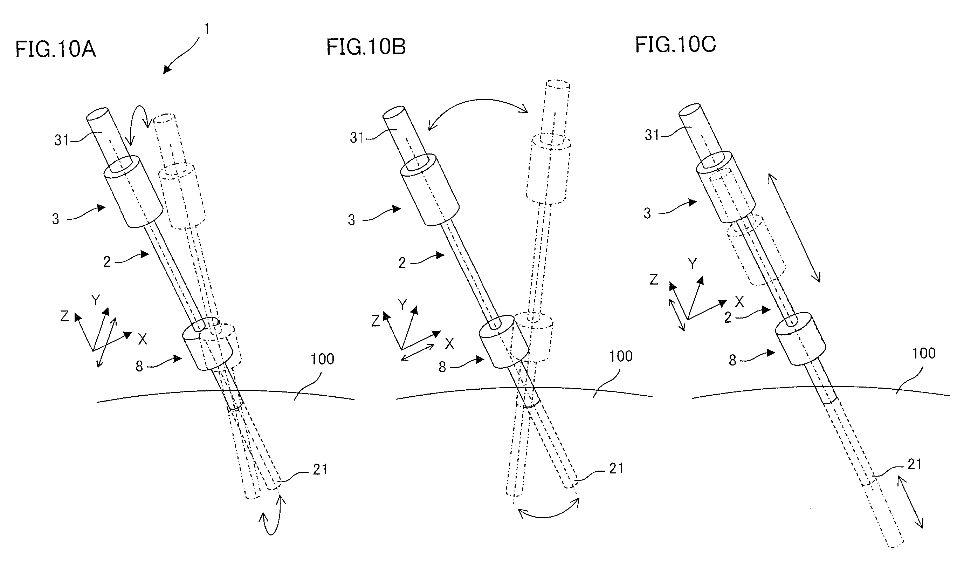

Fig. 10 is a schematic view of operation of the manipulator 1 according to the embodiment of the invention described herein.

In Fig. 10, the XY plane is defined by the drawing sheet plane, and the Z direction is defined by a direction toward the back of the drawing sheet. The manipulator 1 is inserted into a body cavity 100 by way of a trocar 8.

As the operator takes a grasp of a grip 31 to move the manipulator 1 in the YZ plane as shown in Fig. 10A, it causes a front end portion 21 of the manipulator 1 to move in the YZ plane within the body cavity 100 with the trocar 8 as center and in a symmetrical relation with respect to the grip 31.

As the operator takes hold of the grip 31 to move the manipulator 1 in the XZ direction as shown in Fig. 10B, it causes the front end portion 21 of the manipulator 1 to move in the XZ plane within the body cavity 100 in a symmetrical relation to the grip 31 with the trocar 8 as center.

As the operator takes hold of the grip 31 to move the manipulator 1 in the Z direction in a state shown in Fig. 10c, it causes the front-end portion 21 of the manipulator 1 to move in the body cavity 100 in the Z direction.

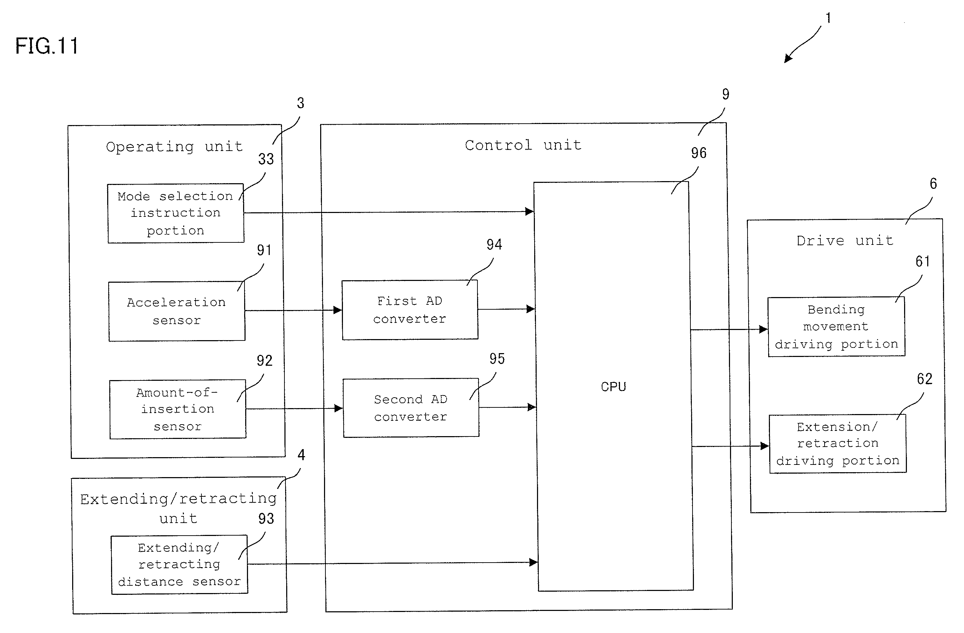

Fig. 11 is a block diagram of the manipulator 1 according to the embodiment of the invention described herein.

The operating unit 3 includes a mode selection instruction portion 33, an acceleration sensor 91, and an amount-of-insertion sensor 92, and the extending/retracting unit 4 includes a moving distance sensor 93. Note here that the acceleration sensor 91 and amount-of-insertion sensor 92 define an amount-of-movement detection sensor.

With a switch such as a button held off, the mode selection instruction portion 33 is in its manual mode in which the extending/retracting movement driving portion 62 is not actuated, and while the button or the like is at the push, it causes the manual mode to be changed over to the electrically operated mode for controlling the extending/retracting movement driving portion 62. The manual and electrically operated modes will be described later.

The acceleration sensor 91 is provided to sense the acceleration of the operating unit 3. As shown in Figs. 10A and 10B, the operator may move the operating unit 3 with the trocar 8 as a fulcrum. By detection of the operating unit 3 during movement, it is possible to detect the position of the front end portion 21.

The amount-of-insertion sensor 92 is provided to detect the distance from the operating unit 3 to the trocar 8 shown in Fig. 10. As shown in Fig. 10C, the operator may move the operating unit 3 relative to the trocar 8 in the insertion or withdrawal direction, and there is a change in that distance during this movement. By detection of such a change, it is possible for the operator to detect the amount of insertion of the insert unit 2, and the position of the front end portion 21 as well.

The extending/retracting distance sensor 93 is provided to detect the moving distance of the extending/retracting unit 4. In the embodiment of the invention described herein, that sensor 93 is used to detect the angle of the first 41b or the second arm 41d with respect to the guide 42 in the extending/retracting unit 4 shown in Fig. 5. With the manipulator 1 here in the electrically operated mode for driving the drive unit 6, the front end portion 21 may be moved without any change in the distance from the operating unit 3 to the trocar 8. By detection of the angle of the first 41b or the second arm 41d with respect to the guide 42 by means of the moving distance sensor 93, it is possible to get the amount of movement of the extending/retracting unit 4 and detect the position of the front end portion 21.

The control unit 9 includes a first AD converter 94, a second AD converter 95, and a CPU 96.

The first AD converter 94 is provided for AD conversion of signals from the acceleration sensor 91, and the second AD converter 95 is provided for AD conversion of signals from the amount-of-insertion sensor 92.

The CPU 96 selects the manual mode or the electrically operated mode in response to a signal from the mode selection instruction portion 33: in the manual mode it does not drive the drive unit 6, and in the electrically operated mode, it controls the drive unit 6 in association with the acceleration sensor 91, amount-of-insertion sensor 92 and extending/retracting distance sensor 93.

Control of the manipulator 1 having such structure is now explained.

One example of the manual mode in which there is no control of the manipulator 1 is first explained.

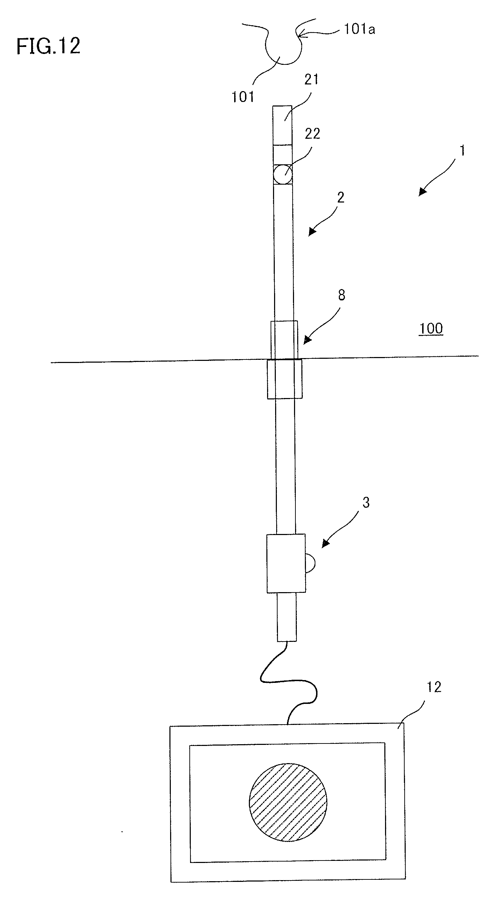

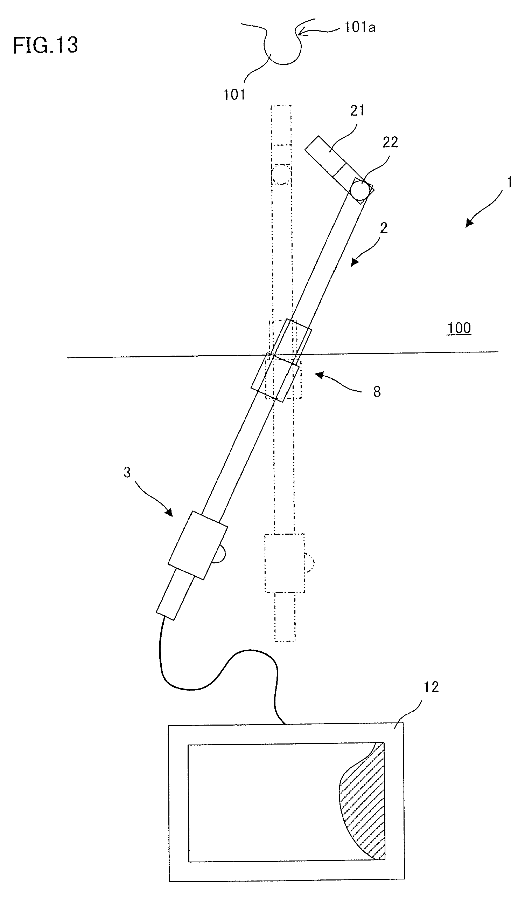

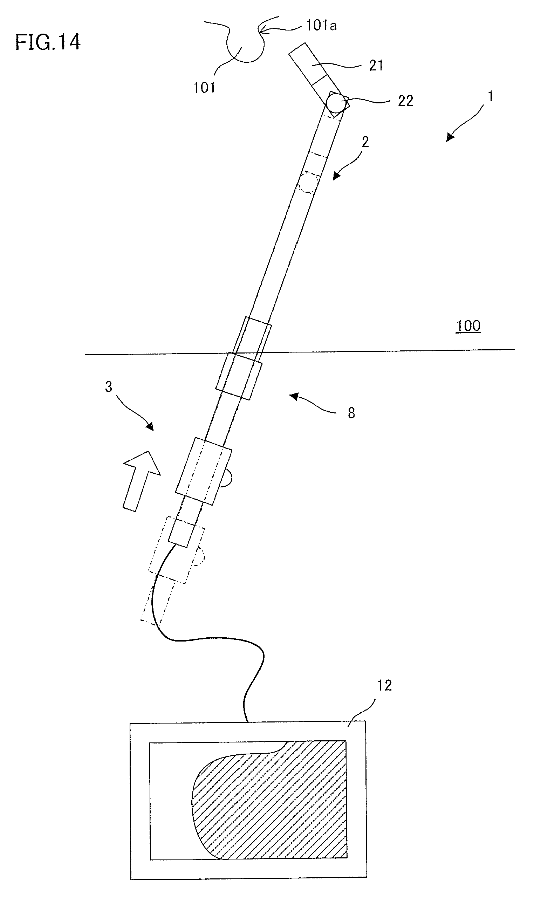

Fig. 12 is a schematic view of a first manual state of the manipulator 1 in the manual mode; Fig. 13 is a schematic view of a second manual state of the manipulator 1 in the manual mode; and Fig. 14 is a schematic view of a third manual state of the manipulator 1 in the manual mode. Note here that the extending/retracting unit 4 is left out of Figs. 12, 13 and 14.

As shown in Fig. 12, a site of interest 101 is observed from the front in the first manual state. Assume here that the operator changes the site 101 of interest to a first site 101a of interest that is positioned at the back of the site 101. The operator must observe the first site 101a while rotating the manipulator 1 at the operating unit 3 with the trocar 8 as center, as can be seen from the second manual state shown in Fig. 13.

In the second manual state, however, the front end portion 21 is away from the site 101, and an image of the site 101 seen on a monitor 17 becomes small while the front end portion 21 deviates from the front of the site 101, ending up with unsatisfactory observation. Here, the operator passes from the second manual state to the third manual state shown in Fig. 14, in which the operating unit 3 can be forced in to bring the front end portion 21 close to the site 101 so that satisfactory images can be well observed.

In the manual mode, an experienced operator could pass swiftly, if instinctively, from the first manual state of Fig. 12 to the third manual state of Fig. 14.

Control of the manipulator 1 in the electrically operated mode is now explained.

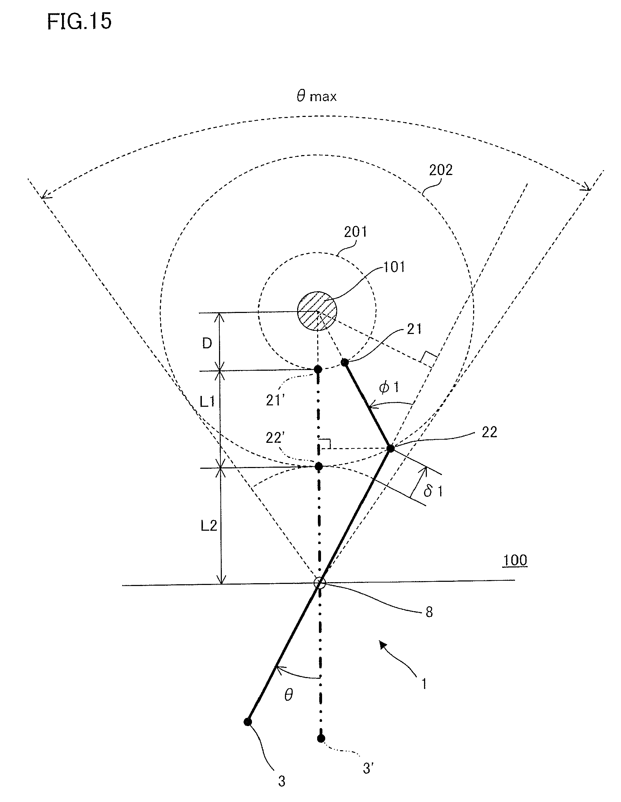

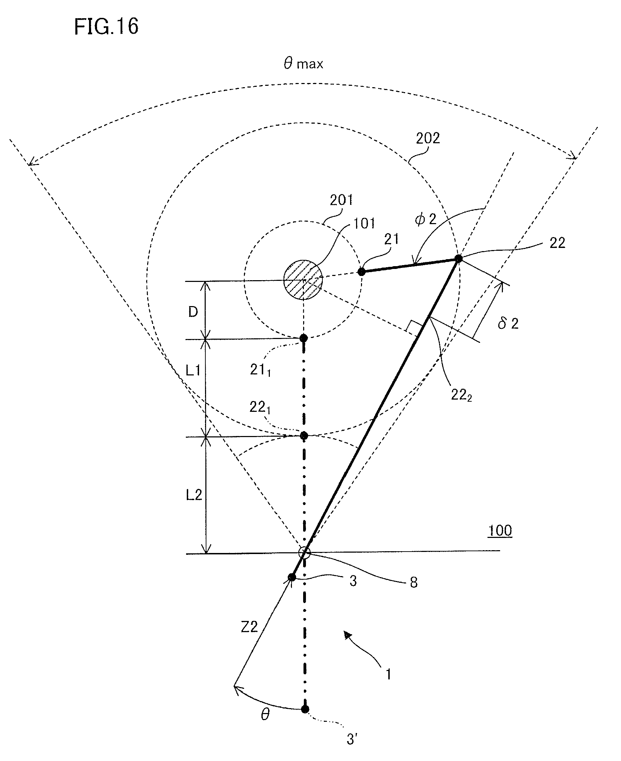

Fig. 15 is descriptive of Equations (1) and (2) used for control of the manipulator 1 here in the electrically operated mode, and Fig. 16 is descriptive of Equations (3) and (4) used for control of the manipulator 1 here in the electrically operated mode. Note here that in Figs. 15 and 16, the front end portion 21 is indicated by a distal point positioned at the foremost end, the bendable portion 22 by an inflection point, the trocar 8 by a pivotal point, and the operating unit 3 by an operation point.

In the electrically operated mode of control of the manipulator 1 described herein, such a manual mode of tasks performed by the operator as shown typically in Figs. 12, 13 and 14 is automated. For instance, control may be performed such that the distance from the front end portion 21 to the site 101 of interest is the same before and after control, and the front end portion 21 directs at the site 101 of interest.

In Fig. 15, the manipulator 1 before control is indicated by a two-dot chain line. Assume now that a distance from the site 101 of interest to a front end portion 21' before control is indicated by D, a distance from the front end portion 21' before control to a bendable portion 22' before control by L1, a distance from the bendable portion 22' before control to the pivotal point for the trocar 8 by L2, and a controllable range by $\theta_{max}$.

In order to make the distance between the front end portion 21 after control and the site 101 of interest the same as the distance before control, the front end portion 21 after control may be located on a first circle 201 having a radius defined by the distance D with the site 101 as center. Further in order to direct the front end portion 21 toward the site 101, the bendable portion 22 after control may be located on a second circle 202 having a radius defined by a distance D+L1 with the site 101 as center. Note here that the controllable range $\theta_{max}$ is defined by an angle made between two tangent lines withdrawn from the trocar 8 to the second circle 202.

Consequently, the amount $\delta1$ of extending/retracting movement that is a controlled variable (amount of control) and the bending angle $\phi1$ may be found from Fig. 15 as mentioned below. However, $\phi1 \leqq 90°$.

$$(D + L1)\sin\phi1 = (D + L1 + L2)\sin\theta$$

$$\phi1 = \sin^{-1}\left(\frac{D + L1 + L2}{D + L1}\sin\theta\right) \qquad (1)$$

$$(L2 + \delta1)\sin\theta = (D + L1)\sin(\pi - \theta - (\pi - \phi1))$$

$$\delta1 = \frac{(D + L1)\sin(\phi1 - \theta)}{\sin\theta} - L2 \qquad (2)$$

A specific case: 90° < $\phi2$ is now explained. This control may be applied to such as when, upon insertion of the manipulator 1, it is impossible for the operator to direct the front end portion 21 toward the site 101 of interest unless the bending angle $\phi$ of the bendable portion 22 is greater than 90°. As is the case with Fig. 15, the amount $\delta2$ of extending/retracting movement that is a controlled variable (amount of control) and the bending angle $\phi2$ may be found from Fig. 16 as mentioned below.

$$(D + L1)\sin(\pi - \phi2) = (D + L1 + L2)\sin\theta$$

$$\phi2 = \pi - \sin^{-1}\left(\frac{D + L1 + L2}{D + L1}\sin\theta\right) \qquad (3)$$

$$(L2 + \delta2)\sin\theta = (D + L1)\sin(\pi - \theta - (\pi - \phi2)$$

$$\delta2 = \frac{(D + L1)\sin(\phi1 - \theta)}{\sin\theta} - L2 \qquad (4)$$

It is here to be appreciated that 90° $<\phi2$ indicates that the acceleration detected by the acceleration sensor 91 shown in Fig. 11, the rate of insertion found by integration of that acceleration, the amount of insertion Z2 detected by the amount-of-insertion sensor 92, etc. may optionally exceed predetermined thresholds. Higher rates of insertion or an increased amount of insertion Z2 imply that the operator desires to observe the site 101 of interest from the back. The predetermined thresholds may be previously stored in a storage (not shown) or the like or, alternatively, they may be set for each control depending on the situations of the site 101.

While the embodiment of the invention here has been explained with reference to two-dimensional movements for the sake of an easy understanding of the invention, it is to be noted that the same may hold true for three-dimensional movements. For three-dimensional movements, the first 201 and the second circle 202 may be replaced by spheres for consideration.

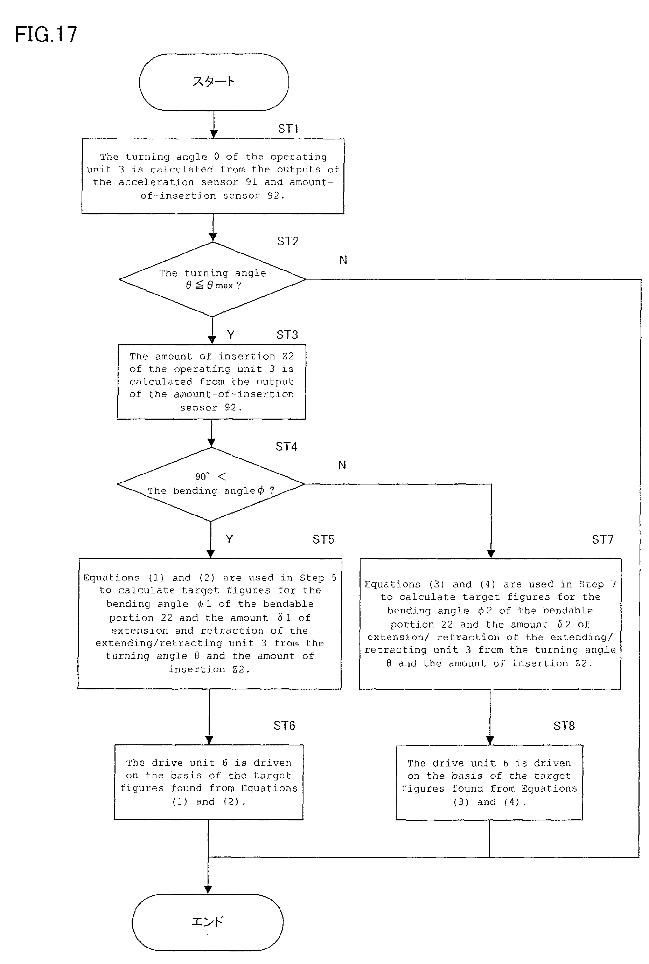

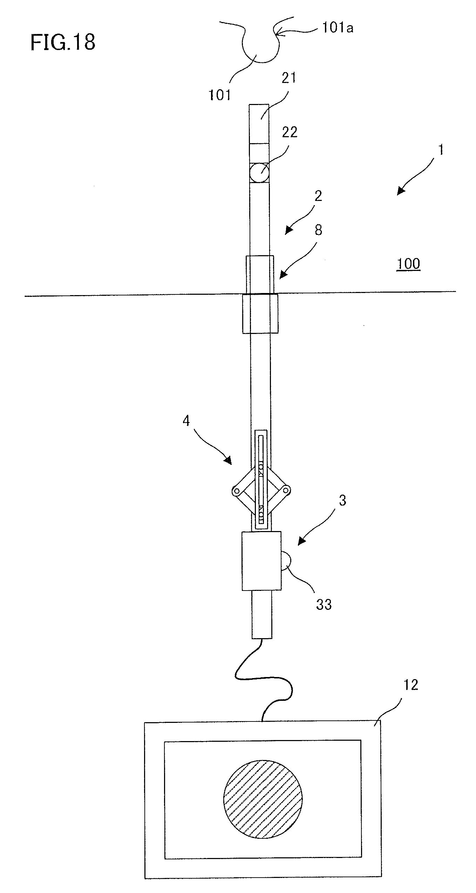

Fig. 17 is a control flowchart for the manipulator 1 described herein, and Fig. 18 is a schematic view of the manipulator 1, described herein, in a state before the electrically operated mode of control is applied to it.

Suppose now that, as shown in Fig. 18, the insert unit 2 of the manipulator 1 has already been inserted and placed in the body cavity 100 inside the skin, and that the endoscope in the manipulator 1 has taken images of the site 101 of interest. The electrically operated mode of control starts in response to an instruction from the mode selection instruction portion 33.

In Step 1, the turning angle $\theta$ of the operating unit 3 is calculated from the outputs of the acceleration sensor 91 and amount-of-insertion sensor 92 (ST1).

In Step 2, whether or not the turning angle $\theta$ found in Step 1 satisfies $\theta \leqq \theta_{max}$ is determined (ST2).

In Step 2, when $\theta \leqq \theta_{max}$ is not satisfied, the electrically operated mode is finished. When the electrically operated mode is finished, an operator should be informed of that the angle is out of a controllable range by warnings.

In Step 2, when $\theta \leqq \theta_{max}$ is satisfied, the amount of insertion Z2 of the operating unit 3 is calculated from the output of the amount-of-insertion sensor 92 (ST3).

In Step 4, whether or not the bending angle $\phi$ of the bendable portion 2 of the manipulator 1 is smaller than 90° is then determined (ST4).

When the bending angle $\phi$ of the bendable portion 2 of the manipulator 1 is found to be smaller than 90° in Step 4, Equations (1) and (2) are used in Step 5 to calculate target figures for the bending angle $\phi1$ of the bendable portion 22 and the amount $\delta1$ of extension and retraction of the extending/retracting unit 3 from the turning angle $\theta$ and the amount of insertion Z2 (ST5).

The operation then goes to Step 6 in which the drive unit 6 is driven on the basis of the target figures found from Equations (1) and (2) (ST6). Note here that bending movement by the driving portion 61 for bending movement and extension/retraction by the driving portion 62 for extension/retraction may take place in any desired order.

When the bending angle $\phi$ of the bendable portion 2 of the manipulator 1 is found to be greater than 90° in Step 4, Equations (3) and (4) are used in Step 7 to calculate target figures for the bending angle $\phi2$ of the bendable portion 22 and the amount $\delta2$ of extension/ retraction of the extending/retracting unit 3 from the turning angle $\theta$ and the amount of insertion Z2 (ST7).

The operation then goes to Step 8 in which the drive unit 6 is driven on the basis of the target figures found from Equations (3) and (4) (ST8). Note here that bending movement by the driving portion 61 for bending and extension/retraction by the driving portion 62 for extension/retraction may take place in any desired order.

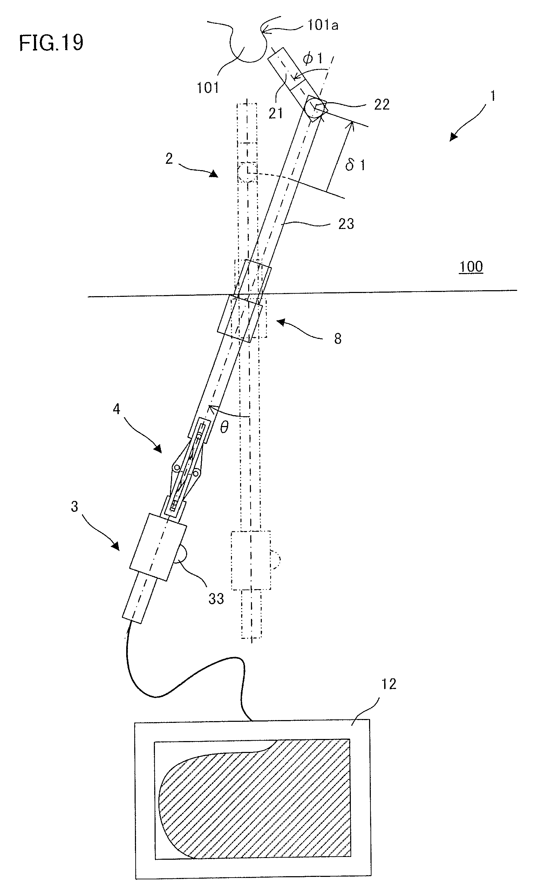

Fig. 19 is a schematic view of the manipulator 1 of the invention described herein just after controlled in the electrically operated mode.

In the manipulator 1 after controlled in the electrically operated mode, for instance, the bendable portion 22 rotates by the bending angle $\phi1$ and the extending/retracting unit 3 moves by the amount of movement $\delta1$ on the basis of the target figures found from Equations (1) and (2), as shown in Fig. 19. As a result, an image of the first site 101a of the site 101 of interest shows up on the monitor 12 on an enlarged scale.

The conventional manipulator is very cumbersome because there is the need of performing separate operations for the position to which the manipulator is to be moved and the angle (posture) with respect to the site of interest. However, the manipulator 1 according to the embodiment of the invention described herein is easy to operate because the task to be performed by the operator is only to align the front end of the manipulator 1 with the site 101 of interest so that automated angle (posture) alignment of the manipulator 1 is achievable by the acceleration sensor 91 or amount-of-insertion sensor 92.

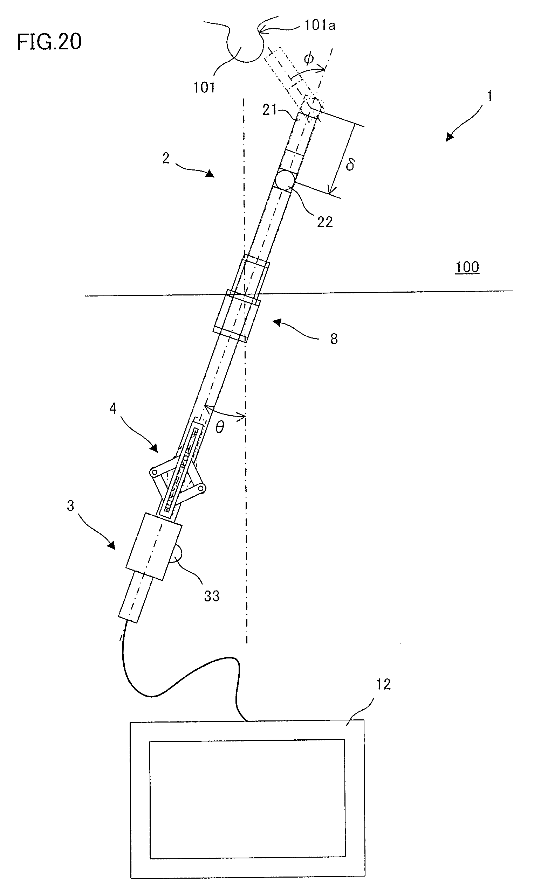

Fig. 20 is a schematic view of the manipulator 1 of the invention just after completion of the electrically operated mode.

After controlled in the electrically operated mode, that mode is cleared by the operator in response to the mode selection instruction portion 33. Here if the operator judges from the output of the acceleration sensor 91 or amount-of-insertion sensor 92 that the manipulator 1 moves a given distance in the withdrawal direction, the manipulator 1 is actuated such that the bending angle $\phi 1$ of the bendable portion 22 or the amount of extension and retraction $\delta 1$ of the extending/retraction unit 4 are restored to the original conditions, as shown in Fig. 20, and goes back to the linear state. Then, the operator may withdraw the manipulator 1 in the linear state from within the body cavity.

Note here that bending movement by the driving portion 61 and extension/retraction by the driving portion 62 may take place in any desired order. Preferably, bending movement by the driving portion 61 and extension/retraction by the driving portion 62 should take place with the images of the site 101 of interest showing up on the screen.

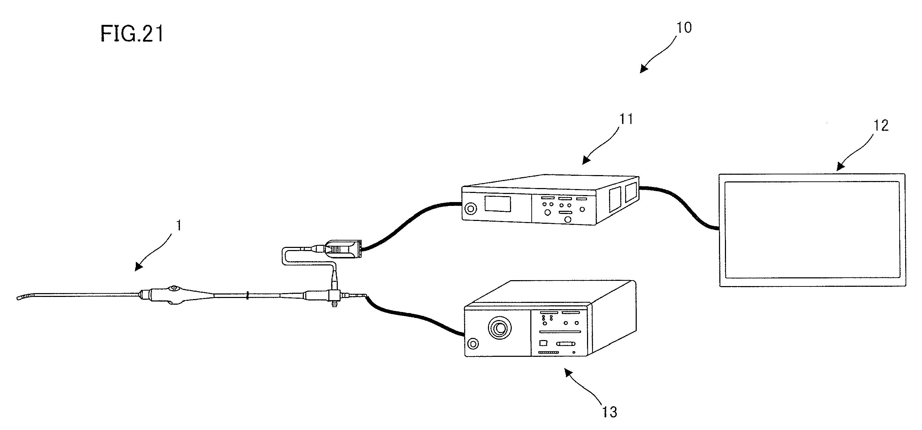

Fig. 21 is a schematic view of the manipulator system 10 described herein.

The manipulator system 10 here comprises a manipulator 1 including an endoscope as described above, an image processor 11, a monitor 12, and a light source device 13.

The image processor 11 is provided to subject image signals from the endoscope in the manipulator 1 to a variety of image processing such as Y control, edge enhancement and output format conversion and send them out to the monitor 12. The monitor 12 presents the image signals received from the image processor 11 as images for observation. The light source device 13 is provided to generate illumination light from an illumination portion 21c of the front-end portion 21 shown in Fig. 2 through a light guide fiber (not shown) in the manipulator 1, and illuminates the site.

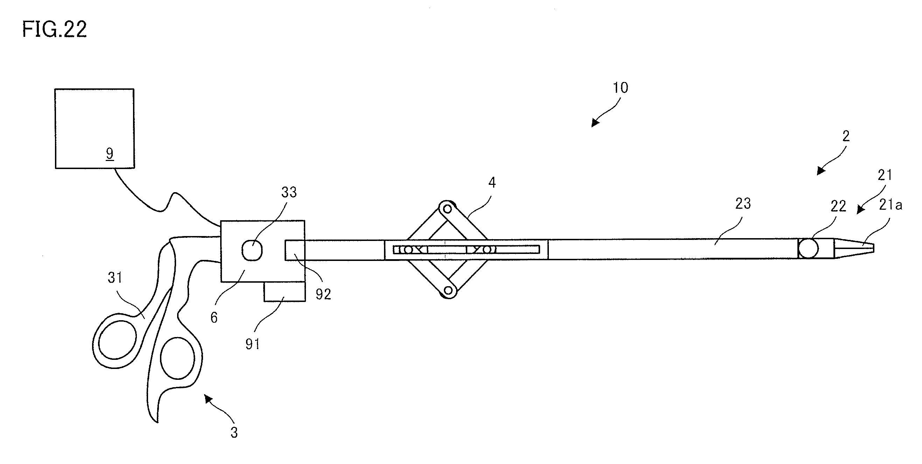

Fig. 22 is a schematic view of another embodiment of the manipulator system 10.

A gripping portion 21a is formed of forceps capable of taking a grip of the site 101 of interest and incising it or the like. The gripping portion 21a may be opened or closed by activation of a wire (not shown) by the operating unit 3 like scissors. This system is otherwise the same as the manipulator 1 including an endoscope. Note here that the gripping portion 21a may be provided separately from the operating unit 3 for wireless remote control or the like.

It is to be appreciated that the invention is not limited to some embodiments described herein. The explanation of some specific embodiments includes many detailed matters for the sake of illustration; however, it would be obvious to those skilled in the art that even when various variations and changes are applied to these detailed matters, they do not depart from the scope of the invention. Thus, the illustrative embodiments of the invention are described without a loss of the generality of what is claimed and without any limitation on what is claimed.

IDUSTRIAL APPLICABILITY

The manipulator according to the invention ensures that less experienced operators can be relieved of uncomfortableness because there is no shifting of the field of vision upon forced insertion into the body cavity or withdrawal.

EXPLANATION OF THE REFERENCE NUMERALS

| | |
|---|---|
| 1: | Manipulator |
| 2: | Insert unit |
| 21: | Front-end portion |
| 22: | Bendable portion |
| 23: | Power transmission portion |
| 3: | Operating unit |
| 31: | Grip |
| 32: | Main body of the operating unit |
| 33: | Mode selection instruction portion |
| 34: | Coupling |
| 4: | Extending/retracting unit |
| 41: | Link |

42:     Guide
5:     Wire
51:     First bendable-portion activation wire
52:     Second bendable-portion activation wire
53:     Link activating wire
6:     Drive unit
61:     Bending movement driving portion
62:     Extension/retraction driving portion
8:     Trocar
9:     Control unit
91:     Acceleration sensor
92:     Amount-of-insertion sensor
93:     Extending/retracting distance sensor
94:     First AD converter
95:     Second AD converter
96:     CPU
10:     Manipulator system
11:     Image processor
12:     Monitor
13:     Light source device
100:     Body cavity
101:     Site of interest

**Claims**

1.   A manipulator, including:

   an insert unit capable of being inserted through the body cavity,
   an operating unit for operating the insert unit,
   an extending/retracting unit for moving the insert unit forward or backward,
   a drive unit for driving the insert unit and the extending/retracting unit, and
   a control unit for controlling the drive unit in association with operation of the operating unit, **characterized in that**:

      the insert unit includes a bendable portion, and
      the drive unit includes a bending movement driving portion for generating driving force for bending the bendable portion, and an extension/retraction driving portion for generating driving force for moving the extending/retracting unit forward or backward, wherein:

         the control unit controls the extension/retraction driving portion and the bending movement driving portion in association with operation of the operating unit.

2.   A manipulator as recited in claim 1, where the operating unit further includes a mode selection instruction portion for selecting either one of a manual mode, and a control mode in which the operating unit controls the drive unit in association with operation of the operating unit.

3.   A manipulator as recited in claim 1 or 2, wherein the operating unit includes an amount-of-movement sensor capable of detecting an amount of movement of the operating unit, and
   the control unit controls the drive unit in association of an amount of movement of the operating unit detected by the amount-of-movement sensor.

4.   A manipulator as recited in claim 3, wherein the control unit performs control such that the front-end portion after controlled directs at the site of interest.

5.   A manipulator system, **characterized by** comprising:

   a manipulator as recited in any one of claims 1 to 4 and including an endoscope,
   an image processor for applying image processing to image signals obtained from the endoscope, and

a monitor for displaying image signals sent from the image processor.

6. A method for controlling a manipulator including:

an insert unit capable of being inserted in the body cavity,
an operating unit for operating the insert unit, and
an extending/retracting unit for moving the insert unit forward or backward, **characterized by** comprising steps of:

calculating a turning angle of the operating unit,
calculating an amount of insertion of the operating unit, and
controlling a bending angle of a bendable portion included in the insert unit and an amount of extension/retraction of the extending/retracting unit in association with the turning angle and amount of insertion of the operating unit.

7. A method for controlling a manipulator as recited in claim 6, wherein the step of controlling the bending angle of the bendable portion and the amount of extension/retraction of the extending/retracting unit is performed such that the front-end portion after controlled directs at the site of interest.

FIG. 1

FIG.2

FIG.3

EP 2 979 609 A1

FIG.4

FIG.5

FIG.6A

FIG.6B

FIG.7A

FIG.7B

FIG.8A

FIG.8B

FIG.9A

FIG.9B

FIG.10A    FIG.10B    FIG.10C

EP 2 979 609 A1

FIG.11

1

Drive unit 6
- Bending movement driving portion 61
- Extension/retraction driving portion 62

Control unit 9
- CPU 96
- First AD converter 94
- Second AD converter 95

Operating unit 3
- Mode selection instruction portion 33
- Acceleration sensor 91
- Amount-of-insertion sensor 92

Extending/retracting unit 4
- Extending/retracting distance sensor 93

FIG.12

FIG.13

FIG.14

FIG.15

FIG.16

## FIG.17

FIG.18

FIG.19

FIG.20

FIG.21

FIG.22

2

10

21
21a
22
23

4

102 101

33
92
91
6

9

31
3

## INTERNATIONAL SEARCH REPORT

International application No.

PCT/JP2014/051200

A. CLASSIFICATION OF SUBJECT MATTER
*A61B1/00*(2006.01)i, *G02B23/24*(2006.01)i

According to International Patent Classification (IPC) or to both national classification and IPC

B. FIELDS SEARCHED

Minimum documentation searched (classification system followed by classification symbols)
A61B1/00, G02B23/24

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2009-18116 A (Olympus Medical Systems Corp.), 29 January 2009 (29.01.2009), paragraphs [0011] to [0051]; fig. 1 to 5 (Family: none) | 1-5 |

☐ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 February, 2014 (06.02.14) | 18 February, 2014 (18.02.14) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2014/051200

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 6, 7
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2014/051200

Continuation of Box No.II-1 of continuation of first sheet(2)

Claims 6 and 7 include a method for inserting an insertion part of an endoscope into a body cavity (human small or large intestine) and therefore pertain to methods for treatment of the human body by surgery. Thus, these claims relate to a subject matter which this International Searching Authority is not required, under the provisions of Article 17(2)(a)(i) of the PCT and Rule 39.1(iv) of the Regulations under the PCT, to search.

Form PCT/ISA/210 (extra sheet) (July 2009)

**EP 2 979 609 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005074148 A